# EUROPEAN PATENT SPECIFICATION

(11) **EP 3 216 429 B1**
(45) Date of publication and mention of the grant of the patent: **22.04.2020**
(21) Application number: 17159864.2
(22) Date of filing: 08.03.2017
(51) Int. Cl.: A61F 5/01

(54) **AN ORTHOPEDIC ORTHOSIS OR BRACE DESIGNED FOR REHABILITATION OF JOINTS OF THE HUMAN BODY**
ZUR REHABILITATION VON GELENKEN DES MENSCHLICHEN KÖRPERS DESIGNTE ORTHOPÄDISCHE ORTHESE ODER STÜTZE
ORTHÈSE OU ATTELLE ORTHOPÉDIQUE CONÇUE POUR LA RÉHABILITATION D'ARTICULATIONS DU CORPS HUMAIN

(30) Priority: 11.03.2016 IT UA20161583
(43) Date of publication of application: 13.09.2017
(73) Proprietor: F.G.P. S.R.L., 37062 Villafranca Di Verona (Verona) (IT)
(72) Inventor: FERRIGOLO, Moreno, 37062 Villafranca di Verona (IT); TURRINI, Alberto, 37062 Villafranca di Verona (IT)
(74) Representative: Sandri, Sandro

(56) References cited:
- WO-A1-86/04228
- US-A1- 2006 155 230
- US-A1- 2013 041 300

## Description

### FIELD OF APPLICATION

The present invention relates to a joint for restraining arches of orthopedic orthoses, i.e. for restraining arches of knee braces for osteoarthritis or other similar orthopedic orthoses designed for rehabilitation of joints of the human body, such as the elbow, the ankle, the shoulder or the like.

The present solution envisages providing an orthosis endowed with a number of features serving to impart elasticity to the restraining arches through the use of elastic type joints enabling a flexible anchorage of the orthosis to the thigh or leg portions of the limb to which the orthosis is fitted.

In particular, the restraining arches that enable the anchorage of the orthosis to the limb to be treated, according to the invention, are fitted to the frame of the orthosis by means of the interposition of elastic type elements, which permit the arch to make small, limited oscillations in relation to muscular deformation during walking.

The present invention has application in the medical and orthopedic fields, in particular in the manufacture of orthoses in general, as well as prostheses and braces mainly for use in conservative, post-traumatic, rehabilitative and postoperative therapy.

### PRIOR ART

It is well known that individuals who have orthopedic problems with knee joints, as well as other joints, such as the ankle or elbow, above all in the case of lesions due to injury or as a consequence of a previous surgical procedure, need to use an orthopedic orthosis, or brace, designed to ensure or control the function of a hinged restraint between the femur and tibia or other lever pivot points of joints in general, supporting stresses that would otherwise be damaging for the joint itself.

The function of an orthosis is, in general, to ensure a relative immobilization or limitation of a joint affected by injuries or arthritis or ligament sprains or which has undergone a surgical procedure.

Another use of an orthosis is in conjunction with functional rehabilitation or re-education, where the orthosis can be used to reduce the load on a joint and decrease pain, or be adopted for preventive purposes in cases of osteoporosis or bone yielding.

An orthosis usually consists of a rigid or soft frame encircling the limb and designed to ensure an adequate harnessing of the joint with the aim of preventing the occurrence of stress on the ligaments or synovial membranes when the injured and/or convalescing individual is walking.

Orthoses for joints in general and for the knee joint in particular consist of a rigid frame designed to ensure an adequate harnessing of the joint with the aim of preventing the occurrence of stress on said joint and limiting or preventing torsion during walking.

The frame of classic orthoses, taking into consideration the case of the knee joint, comprises means of restraint to the femur and tibia in areas close to the knee and a structure connecting said means with a hinge positioned at the level of knee itself.

In order to assure sufficient freedom of movement of the limb, the frame extends mostly at the sides of the knee to allow reciprocal oscillation between the femur and tibia.

The means of restraint usually consist of Velcro straps, particularly suitable for adjusting the fastening tension; the straps are positioned on special fixed supports in such a way as to wrap around the body areas belonging to the joint involved.

More particularly, the means of restraint of the orthoses to the limb comprise at least two arches of a substantially rigid type positioned at either end of the side uprights and solidly joined to the latter, i.e. constituting extensions thereof which are connected to form an arch, and a series of flexible type anchoring straps positioned on the uprights themselves.

Even more particularly, the restraining arch associated with the upper uprights is generally positioned towards the front part of the thigh, in such a way as to contain thrusts from the front side, whereas the restraining arch associated with the lower uprights is positioned in the rear part of the leg, i.e. resting close to the calf, in such a way as to contain thrusts from the rear side.

The anchoring straps of the orthosis are positioned in a complementary manner relative to the restraining arches, so that their presence completes the restraint of the orthosis by completely wrapping around the limb. Additional straps are located in the intermediate areas of the uprights, in such a way as to provide the maximum hold in wrapping around the leg, whereas the hinged joint that connects the side uprights enables the controlled flexion of the leg at the knee.

The use of these types of uprights has brought to light a problem which limits the correct functioning of the orthosis due to the rigidity of the arches solidly joined to the uprights positioned in front of the thigh part and behind the calf, resulting in a cutting sensation provoked by such known arches, which are normally rigid and integral with the structure.

It has been found, in fact, that the musculature of the leg and the flexion of the limbs relative to the knee provoke a lengthening or shortening of the distance between the anchoring straps of the orthosis and the pivot at the knee, which acts as a centre of rotation, provoking anomalous traction and a cutting sensation in the area in which the orthosis rests against the skin, with consequent discomfort for the user, who is sometimes even forced to manually correct the position of the orthosis.

In fact, the traction exerted on the anchoring straps of the orthosis by the muscle fascia of the leg during the flexion movements thereof, in addition to the formation of anomalous tensions and cutting sensations, can also cause the orthosis to come out of place, with all the negative consequences for the overall positioning on the limb of the individual who is wearing it.

Document US 2013/0041300 A1 discloses a knee orthosis having two vertical struts positioned on opposed sides of a knee joint in a generally parallel relationship. Each vertical strut has a hinge member located at a general middle portion; each vertical strut has a top and bottom end portion. An upper thigh cuff attaches at opposed ends to the upper end portions of the two vertical struts whereas a lower shin cuff attaches at opposed ends to the lower end portions. The lower shin cuff has a pair of hinges located proximal to the shin cuff opposed ends for permitting the shin cuff to pivot downwardly away from a shin of a person wearing the knee orthosis.

### DESCRIPTION OF THE INVENTION

The present invention aims to provide a joint for restraining arches of orthoses which is capable of eliminating or at least reducing the drawbacks highlighted above.

The invention aims in particular to provide an elastic type joint for restraining arches of orthopedic orthoses, or other similar orthopedic braces, whose use enables the restraining arches to remain partially disengaged from the respective supports of the uprights, instead of being solidly joined thereto, to permit a controlled shifting of the thrusts using the same straps which retain the orthosis.

It has in fact been found that when the solution according to the invention is used, the thrusts exerted by the muscle fascia of the individual who is wearing the orthosis are absorbed both by the restraining arches and the anchoring straps, which can shift angularly in an elastic manner, while preserving the positioning and simultaneous restraint of the orthosis to the thigh and calf without shifting.

This is achieved by means of a joint for restraining arches of orthoses provided with elastic type joints, whose features are described in the main claim.

The dependent claims of the solution in question outline advantageous embodiments of the invention.

The main advantages of this solution substantially regard the possibility for the orthosis to elastically absorb the traction that the musculature exerts during flexion on the restraining arches and on the anchoring straps associated with them.

In other words, the elasticity of the restraining arches of the orthosis according to the invention prevent the cutting sensations of the known arches - which are normally rigid, or integral with the structure - with all the advantages deriving therefrom.

### ILLUSTRATION OF THE DRAWINGS

Other features and advantages of the invention will become more apparent on reading the following description of an embodiment of the invention, provided by way of non-limiting example with the aid of the drawings illustrated in the attached figures, in which:
- figure 1 represents a schematic view showing an orthopedic orthosis or brace in its entirety and the portions of anchorage thereof to the limb of the individual that is wearing it;
- figure 2 is an exploded view of the components of the same orthosis which highlights, in particular, the elastic type system of the restraining arches;
- figure 3 shows a detailed view, according to detail "A" of figure 2, of the joint which, according to the invention, enables the elasticity of the restraining arches relative to the uprights of the orthosis.

### DESCRIPTION OF AN EMBODIMENT OF THE INVENTION

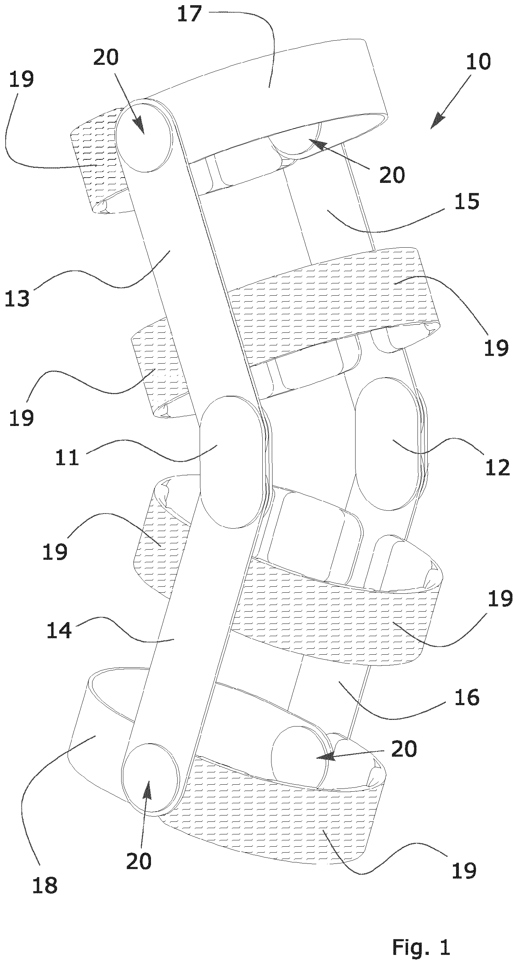

Making reference initially to figure 1, the number 10 denotes in its entirety an orthosis which, in the case illustrated, is designed for the knee, bearing in mind that the same system that will be described can also be applied to other orthoses for joints, such as the elbow, the ankle or others.

The orthosis 10 comprises a pair of hinged joints 11 and 12 with controllable angular travel, wherein each hinged joint acts as a support for respective pairs of femoral and tibial uprights, respectively the uprights 13, 14 for the hinged joint 11 and uprights 15, 16 for the hinged joint 12.

The uprights 13, 14, 15 and 16 act as means of restraint of the orthosis to the limb; these comprise two arches 17 and 18 of a substantially rigid type positioned at either end of the side uprights and solidly joined to the latter, i.e. constituting extensions thereof which are connected to form an arch, associated with a series of flexible anchoring straps 19 positioned on the uprights themselves.

The restraining arches 17 and 18 bring about a stable positioning of the orthosis, lending it the conformation of a substantially tubular frame suitable for fitting to the limb of the individual who must wear it.

As may be seen in figure 1, the restraining arch 17 associated with the upper uprights 13 and 15 is positioned toward the front part of the thigh, in such a way as to contain thrusts from the front side, whereas the restraining arch 18 associated with the lower uprights 14 and 16 is positioned in the rear part of the leg, i.e. resting close to the calf, in such a way as to contain the thrusts from the rear side.

The anchoring straps 19 of the orthosis are positioned in a complementary manner relative to the restraining arches 17 and 18, so that their presence completes the restraint exerted by the arches of the orthosis by wrapping completely around the limb.

Additional straps 19 are located in the intermediate areas of the uprights, in such a way as to provide the maximum hold in wrapping around the leg, whereas the hinged joint that connects the side uprights enables the controlled flexion of the leg at the knee.

According to the invention, it is envisaged that the restraining arches 17 and 18, which are part of the main structure or frame of the orthosis, are fitted to the respective uprights by means of the interposition of a joint denoted in its entirety with the number 20, which allows the arches to remain restrained to the uprights while ensuring a certain rigidity of the frame, but with the possibility of carrying out small angular oscillations, thus enabling the adaptation of the orthosis during flexions of the limb and absorbing the traction exerted by the musculature on the restrained areas.

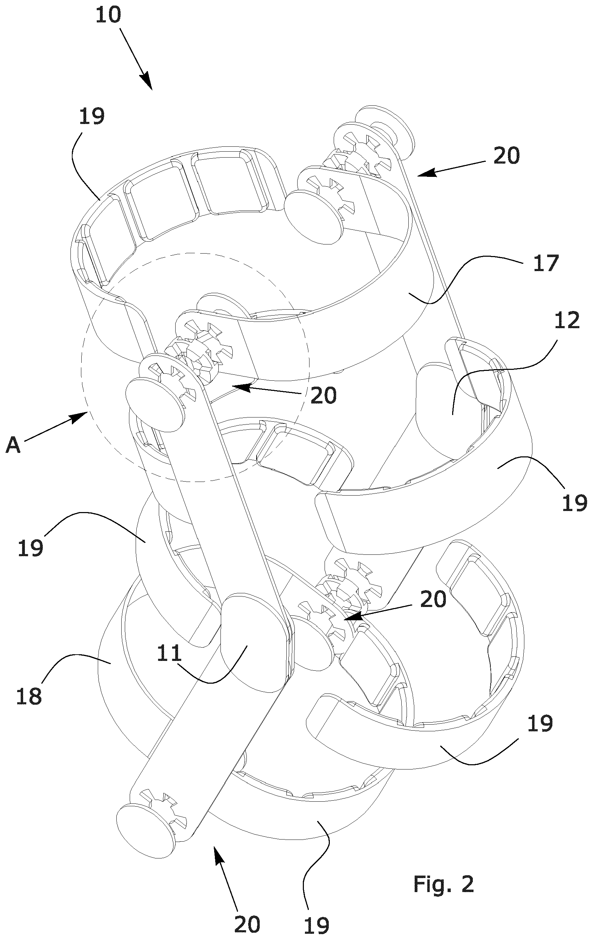

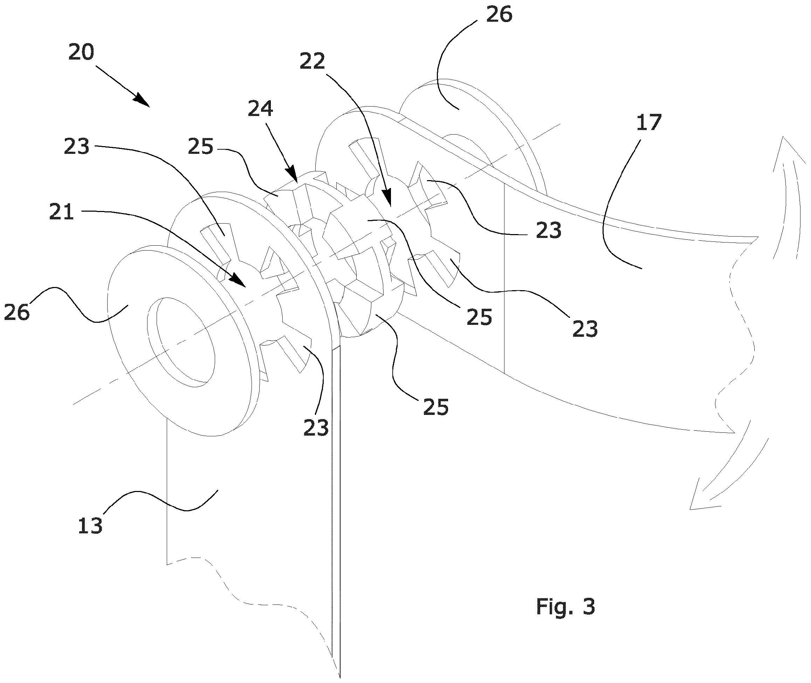

With reference to figure 2 and the detailed figure 3, the joint 20 comprises housings 21 and 22 respectively positioned at the ends of each upright and at the ends of each restraining arch, each of the housings comprising shaped slots 23 which represent restraining elements for an elastic connection element 24, or flexible coupling, positioned between the housings.

The elastic connection element 24 is made from a semi-flexible material such as rubber, or from polyurethane or silicone, and has a substantially ring-like shape provided with a plurality of shaped protuberances 25 that protrude symmetrically with respect to the centre-line on the circular plane of the ring-like body.

The number of protuberances and their shape corresponds to the number and shape of the shaped slots 23 cut in the housings 21 and 22, which enables the reciprocal insertion of the elastic connection element 24 in the housings 21 positioned at the ends of each upright associated with each restraining arch.

The shaped protuberances 25 protrude outwards from the respective housings in which they are inserted, enabling fixing elements 26 to be fitted on them in order to restrain all the components of the joint 20.

It is now clear that the elastic connection element 24 determines a semi-elastic connection such as to ensure a sufficiently stable positioning of the frame made up of the uprights restrained by the hinges and restraining arches while maintaining a slight elasticity, in a rotational direction, of the restraining arches relative to the uprights.

The joint 20 according to the invention, which elastically restrains the parts, is made from an elastic material, i.e. rubber or polyurethane or silicone, with a density that is pre-established according to the elasticity that it is desired to obtain, or else it consists of a spring-type mechanical joint, analogous to a mechanical flexible coupling.

From an operational viewpoint, the orthosis is applied on the limb to be treated so that the hinged joints 11 and 12 are positioned at the height of the knee and the restraining arches 17 and 18 are positioned respectively in the front part of the thigh and behind the calf, and the whole is secured in place using traditional straps 19, which can generally be fixed with Velcro fastenings.

During use of the orthosis, and particularly during walking, the restraining arches can carry out slight angular shifts along the axis of their joint 20 positioned at the ends of the uprights, following the movements of the musculature, so that the cutting sensations perceived with traditional rigid arches are avoided.

According to a further embodiment, the joint for the restraining arches 17 and 18 is also fitted with the respective straps which can likewise rotate to compensate for the muscular movements of the areas opposite those of the semi-elastic arches, further improving the comfort and stability of the orthosis.

The invention has been described above with reference to a preferred embodiment thereof. However, it is clear that the invention is susceptible of numerous variants falling within the scope thereof, within the framework of technical equivalences.

In this regard, an embodiment of a knee brace has been illustrated in the description, but the same system can similarly be applied to orthoses for other joints of the human body.

Similarly, the conformation of the elastic connection element 24 can be any whatsoever, as can the number and conformation of the protuberances and respective housings, according to parameters that can also be established based on the result to be obtained.

## Claims

1. A joint (20) for coupling together respective ends of a substantially rigid restraining arch or cuff (17, 18) and an upright or strut (13-16) of a frame of an orthopedic rehabilitation orthosis (10), such as a knee, elbow or ankle orthosis , whereby each respective restraining arch or cuff (17, 18) end is hinged to a corresponding end of an upright or strut (13-16),
**characterised in that**
the end of said arch or cuff (17, 18) and the end of said upright or strut (13-16) belonging to said joint (20) are provided, each, with a respective housing (21, 22), **in that** each respective housing comprises shaped slots (23), and **in that** a substantially ring-shaped elastic connection element (24) comprising a series of protuberances (25) protruding symmetrically outwards with respect to the centre-line on the circular plane of the ring-like body of said ring-shaped elastic connection member is positioned between said respective housings (21, 22), whereby said protuberances (25) are inserted inside of said shaped slots (23).

2. A joint (20) according to claim 1, **characterised in that** said elastic connection element (24) is made from an elastic material such as rubber, or from polyurethane or silicone.

3. A joint according to any one of claims 1-2, **characterised in that** the number of said protuberances (25) and their shape corresponds to the number and shape of the slots (23) cut in the housings (21, 22), determining the reciprocal insertion of the elastic connection element (24) in the housings (21) positioned at the ends of each upright or strut associated with each restraining arch or cuff.

4. An orthopedic rehabilitation orthosis such as a knee, elbow or ankle orthosis (10) having a pair of hinged joints (11, 12) with controllable angular travel, each hinged joint supporting respective pairs of uprights or struts (13-16), the distal ends of said uprights or struts (13-16) being hinged to respective restraining arches or cuffs (17, 18) by means of respective joints (20),
**characterised in that** each
the joint (20) is a joint (20) according to any of claims 1-3.

5. An orthosis according to claim 4, **characterised in that** said elastic connection element (24) is made from an elastic material such as rubber, or from polyurethane or silicone.

6. An orthosis according to any one of claims 4-5, **characterised in that** the number of said protuberances (25) and their shape corresponds to the number and shape of the slots (23) cut in the housings (21, 22), determining the reciprocal insertion of the elastic connection element (24) in the housings (21) positioned at the ends of each upright or strut (13-16) associated with each restraining arch or cuff (17, 18).

## Patentansprüche

1. Gelenk (20) zum Koppeln jeweiliger Enden eines/r im Wesentlichen starren Beschränkungsbogens bzw. Manschette (17, 18) und einer Aufrechten bzw. Strebe (13-16) eines Rahmens einer orthopädischen Rehabilitationsorthese (10), wie etwa einer Knie-, Ellbogen- oder Knöchelorthese, miteinander, wodurch jedes Ende des/r jeweiligen Beschränkungsbogens bzw. Manschette (17, 18) an einem entsprechenden Ende einer Aufrechten bzw. Strebe (13-16) angelenkt ist,
**dadurch gekennzeichnet, dass**
das Ende des Bogens bzw. der Manschette (17, 18) und das Ende der Aufrechten bzw. Strebe (13-16), die zu dem Gelenk (20) gehören, jeweils mit einem jeweiligen Gehäuse (21, 22) versehen sind, dadurch, dass jedes jeweilige Gehäuse geformte Schlitze (23) umfasst, und dadurch, dass ein im Wesentlichen ringförmiges elastisches Verbindungselement(24), welches eine Reihe von Vorsprüngen (25) umfasst, die in Bezug auf die Mittellinie auf der kreisförmigen Ebene des ringartigen Körpers des ringförmigen elastischen Verbindungselements symmetrisch nach außen vorstehen, zwischen den jeweiligen Gehäusen (21, 22) positioniert ist, wodurch die Vorsprünge (25) im Inneren der geformten Schlitze (23) eingesetzt sind.

2. Gelenk (20) nach Anspruch 1, **dadurch gekennzeichnet, dass** das elastische Verbindungselement (24) aus einem elastischen Material, wie etwa Kautschuk, oder aus Polyurethan oder Silikon hergestellt ist.

3. Gelenk nach einem der Ansprüche 1-2, **dadurch gekennzeichnet, dass** die Anzahl der Vorsprünge (25) und ihre Form der Anzahl und Form der Schnitze (23), die in die Gehäuse (21, 22) geschnitten sind, entsprechen, wobei sie das gegenseitige Einsetzen des elastischen Verbindungselements (24) in die Gehäuse (21) bestimmen, die an den Enden jeder Aufrechten bzw. Strebe positioniert sind, die jedem Beschränkungsbogen bzw. jeder Manschette zugeordnet sind.

4. Orthopädische Rehabilitationsorthese, wie etwa eine Knie-, Ellbogen- oder Knöchelorthese (10), welche ein Paar angelenkter Gelenke (11, 12) mit regelbarem Winkelhub aufweist, wobei jedes angelenkte Gelenk jeweilige Paare Aufrechter bzw. Streben (13-16) trägt, wobei die distalen Enden der Aufrechten bzw. Streben (13-16) an jeweilige Beschränkungsbogen bzw. Manschetten (17, 18) mittels jeweiliger Gelenke (20) angelenkt sind,
**dadurch gekennzeichnet, dass**
jedes Gelenk (20) ein Gelenk (20) nach einem der Ansprüche 1-3 ist.

5. Orthese nach Anspruch 4, **dadurch gekennzeichnet, dass** das elastische Verbindungselement (24) aus einem elastischen Material, wie etwa Kautschuk, oder aus Polyurethan oder Silikon hergestellt ist.

6. Orthese nach einem der Ansprüche 4-5, **dadurch gekennzeichnet, dass** die Anzahl der Vorsprünge (25) und ihre Form der Anzahl und Form der Schnitze (23), die in die Gehäuse (21, 22) geschnitten sind, entsprechen, wobei sie das gegenseitige Einsetzen des elastischen Verbindungselements (24) in die Gehäuse (21) bestimmen, die an den Enden jeder Aufrechten bzw. Strebe (13-16) positioniert sind, die jedem Beschränkungsbogen bzw. jeder Manschette (17, 18) zugeordnet sind.

## Revendications

1. Articulation (20) pour accoupler les extrémités respectives d'un arceau ou d'un brassard de retenue (17, 18) sensiblement rigide et un montant ou un support (13-16) d'un cadre d'une orthèse de rééducation orthopédique (10), telle qu'une orthèse de genou, de coude ou de cheville, selon laquelle chaque extrémité d'un arceau ou d'un brassard de retenue (17, 18) respectif est articulée à une extrémité correspondante d'un montant ou d'un support (13-16), **caractérisée en ce que** l'extrémité dudit arceau ou brassard (17, 18) et l'extrémité dudit montant ou support (13-16) appartenant à ladite articulation (20) sont pourvues, chacune, d'un logement respectif (21, 22), **en ce que** chaque logement respectif comprend des fentes façonnées (23), et **en ce qu'**un élément de connexion élastique (24) sensiblement en forme d'anneau comprenant une série de protubérances (25) faisant saillie symétriquement vers l'extérieur par rapport à la ligne médiane sur le plan circulaire du corps en forme d'anneau dudit élément de connexion élastique en forme d'anneau est positionné entre lesdits logements respectifs (21, 22), selon laquelle lesdites protubérances (25) sont insérées à l'intérieur desdites fentes façonnées (23).

2. Articulation (20) selon la revendication 1, **caractérisée en ce que** ledit élément de connexion élastique (24) est réalisé dans un matériau élastique tel que du caoutchouc, ou dans un polyuréthane ou silicone.

3. Articulation selon l'une quelconque des revendications 1-2, **caractérisée en ce que** le nombre desdites protubérances (25) et leur forme correspondent au nombre et à la forme des fentes (23) découpées dans les logements (21, 22), déterminant l'insertion réciproque de l'élément de connexion élastique (24) dans les logements (21) positionnés aux extrémités de chaque montant ou entretoise associé à chaque arceau ou brassard de retenue.

4. Orthèse de rééducation orthopédique telle qu'une orthèse de genou, de coude ou de cheville (10) ayant une paire d'articulations articulées (11, 12) à course angulaire réglable, chaque articulation articulée supportant des paires respectives de montants ou d'entretoises (13-16), les extrémités distales desdits montants ou entretoises (13-16) étant articulées aux arceaux ou brassards de retenue respectifs (17, 18) par au moyen d'articulations respectives (20) **caractérisée en ce que** chaque articulation (20) est une articulation (20) selon l'une quelconque des revendications 1-3.

5. Orthèse selon la revendication 4, **caractérisée en ce que** ledit élément de connexion élastique (24) est réalisé dans un matériau élastique tel que du caoutchouc, ou dans un polyuréthane ou silicone.

6. Orthèse selon l'une quelconque des revendications 4-5, **caractérisé en ce que** le nombre desdites protubérances (25) et leur forme correspondent au nombre et à la forme des fentes (23) découpées dans les logements (21, 22), déterminant l'insertion réciproque de l'élément de connexion élastique (24) dans les logements (21) positionnés aux extrémités de chaque montant ou entretoise (13-16) associé à chaque arceau ou brassard de retenue (17, 18).
